# EUROPEAN PATENT SPECIFICATION

(11) **EP 2 560 609 B1**
(45) Date of publication and mention of the grant of the patent: **07.09.2016**
(21) Application number: 11721118.5
(22) Date of filing: 22.04.2011
(51) Int. Cl.: A61K 8/99, A61Q 15/00

(54) **COSMETIC USE OF A LYSATE OF BIFIDOBACTERIUM SPECIES FOR TREATING BODY ODOR**
KOSMETISCHE VERWENDUNG VON EINEM BIFIDOBACTERIUM-LYSAT ZUR BEHANDLUNG VON KÖRPERGERUCH
UTILISATION COSMETIQUE D'UN LYSAT DE BIFIDOBACTERIUM POUR TRAITER LES ODEURS CORPORELLES

(30) Priority: 11.05.2010 US 344029 P; 23.04.2010 FR 1053138
(43) Date of publication of application: 27.02.2013
(73) Proprietor: L'Oréal, 75008 Paris (FR)
(72) Inventor: CASTIEL, Isabelle, F-06200 Nice (FR); GUENICHE, Audrey, F-92500 Rueil Malmaison (FR); BERNARD, Dominique, F-92170 Vanves (FR)
(74) Representative: Nony
(86) International application number: PCT/IB2011/051765
(87) International publication number: WO 2011/132176

(56) References cited:
- EP-A1- 1 110 555
- FR-A1- 2 876 029
- FR-A1- 2 912 917

## Description

The present invention is directed mainly toward proposing a novel active agent for preventing and/or treating human body odor.

In general, the human body odor that appears at the skin's surface is liable to change with age, and especially to be exacerbated by a certain number of physiological or nonphysiological phenomena.

Thus, it is known that chronological aging is generally accompanied by an exacerbation of body odor generally inherent to a change in certain biological mechanisms.

It is also known that certain physiological hormonal modifications arising in different age categories, and of nonpathological type (for example puberty or pregnancy), or with regard to particular emotional states (for example stress), may be the cause of undesired body odor.

Similarly, the appearance of this undesired odor may also be brought about and/or stimulated by certain dietary habits. Specifically, it is known that certain foods, for instance garlic, onions, plants of the Brassicaceae family (formerly known as Crucifera), or even certain spices, have the effect, once ingested, of contributing toward the appearance of body odor.

Furthermore, as emerges from the text hereinbelow, a certain number of types of body odor are directly linked to the presence, excessive or otherwise, of bodily fluids secreted in the epidermis, these bodily fluids more particularly being sweat or sebum.

Thus, the change in body odor is especially associated with a quantitative and/or qualitative change in the compounds and/or substances present at the surface of the skin, also known as the "cutaneous substrate", whose degradation by the resident cutaneous microflora is the cause of the biosynthesis of malodorous volatile compounds.

The present invention is precisely directed toward treating and/or preventing all these types of body odor and more particularly that which appears predominantly on the areas of skin where certain bodily fluids are secreted, such as sweat and sebum.

It should be noted that excessive secretion of sweat may also be the cause and/or an aggravating factor in the appearance of this body odor. Such hypersudation, or hyperhydrosis, may especially be associated with nonpathological disorders, for instance an emotional state (stress), the consumption of medicaments and/or alcohol, an endocrine or hormonal dysfunction of nonpathological type (puberty, menopause, pregnancy), age or environmental conditions (heat).

As regards sweat, the physiological function that is its cause, namely perspiration or sweating, occurs to varying degrees in all mammals. In man, the main function of this phenomenon is temperature regulation and the elimination of toxins.

Sweat is a liquid secreted by two types of sweat glands, namely the eccrine glands and the apocrine glands.

The eccrine glands, which are found over the entire body surface, secrete a transparent, aqueous, odorless sweat. This eccrine sweat plays a role in moisturizing the horny layer, in microbiological protection, in gripping and in percutaneous absorption.

For their part, the apocrine glands, the excretory part of which is located in the upper hair follicles, are more particularly localized in the armpits, the auditory canals, the mammary areolae, the perianal region and the feet. These glands do not become functional until puberty, and secrete a viscous sweat, of milky appearance and sparingly odorous. This apocrine sweat is rich in fat and also contains protein. Its secretion is psychically regulated.

In point of fact, sweat, whether it is of eccrine or apocrine origin, and provided that it is not excessively generated, has little or no odor when it is secreted. It is the contact of sweat and more particularly of apocrine sweat with bacteria that induces the formation of volatile and malodorous compounds, via the bacterial degradation of this sweat. The compounds arising from the degradation of sweat and which are the cause of unpleasant odors are especially represented by C₂-C₅ short-chain volatile acids, malodorous steroids, such as the steroid 16-androstene, C₆-C₁₂ fatty acids, such as the trans (E) isomer of 3-methyl-2-hexenoic acid (3M2H), or sulfanylalkanols.

Thus, the axillary region (armpit) is the area of the body where the most bacteria are found (approximately 10⁶ cells/cm²).

As regards sebum, it is secreted by the sebaceous glands, which are generally annexed to a hair. It is especially formed by more than 96% of fats composed of 43% glyceride, 16% free fatty acid, 25% wax (cerumen) and 12% squalene.

The main role of sebum, which is yellowish-white in color and of pasty consistency, is to protect the skin against microbial attack (fungi, bacteria) especially via the presence of lactic acid and fatty acid. It also participates in lubricating the skin by protecting it against moisture and dryness and maintaining the suppleness of the epidermis.

In combination with the sweat and the debris present at the surface of the skin, sebum forms a hydrolipid film that covers the horny layer and thus affords it impermeability. The odor associated therewith usually turns out to be linked to the fact, firstly, that it conveys certain odoriferous molecules, for instance pheromones, and, secondly, that, like sweat, it is liable to undergo bacterial degradation.

For obvious reasons, body odor, and in particular the unpleasant odors as mentioned above, are perceived as being socially invalidating and, in this respect, it is considered important to efficiently control the biological phenomena that generate them.

To combat unpleasant odors, and in particular those linked to perspiration, several compounds have already been proposed, which, by topical application, are capable of inhibiting sweating, of inhibiting the bacteria responsible for the chemical transformations described above, of masking said odors, or of reducing/destroying the indigenous microflora.

In particular, it has generally been proposed to act on the sweat glands by regulating/blocking the perspiration process, to act on the degradation of sweat using enzymatic or nonenzymatic bacteria, or to act on odor emission via the use of fragrances or odor absorbers.

As regards bactericides, the product most commonly used is triclosan (2,4,4-trichloro-2-hydroxydiphenyl ether), which, however, has the drawback of modifying the entire cutaneous microflora and also of being inhibited by certain compounds commonly used in cosmetics. It also has an insoluble nature in water, which does not allow its incorporation into essentially aqueous formulations.

Farnesol (3,7,11-trimethyldodeca-2,5,10-trienol) is also know, but, however, it has the drawback of having allergenic properties.

Another approach consisted in blocking the formation of the volatile fatty acids that are the cause of unpleasant odors by using compounds that are capable of trapping them, of raising the pH to prevent their formation, or alternatively of preventing the proliferation of the bacterial flora.

Yet another approach consisted in decreasing or regulating perspiration by using active substances of antiperspirant type that have the effect of limiting the flow of sweat produced. They generally contain aluminum and/or zirconium salts as active principles.

However, besides the fact that they do not show entirely satisfactory efficacy, all these compounds have the drawback of irritating the skin. In addition, the abovementioned solvents have the disadvantage of modifying the skin's physiology.

Another markedly more extreme solution, which, in this respect, is seldom used, consists of an operation of sympathectomy, excision or curetting type or liposuction of the areas involved in sweat secretion. There is, however, a risk of onset of compensatory phenomena such as the formation of cheloids, a pneumothorax or hyperhydrosis.

Lastly, a final solution consists in acting on the phenomenon of sweating medicinally, especially by injection of toxins, tranquillizers, sedatives/spasmolytics, or anticholinergic agents. The administration of such compounds often results in a substantial number of side effects.

There thus remains a need for novel active agents that are capable of showing efficacy toward body odor, especially that associated directly or indirectly with the presence of bodily fluids secreted on an epidermis, such as sweat and sebum, and which does not have the abovementioned drawbacks.

More particularly, there remains a need for active agents for preventing and/or treating body odor, more particularly that arising from the bacterial degradation of the cutaneous substrate, more particularly including the bodily fluids secreted on an epidermis, such as sweat and sebum, and more particularly apocrine sweat, on areas of the human body that are subject to heavy sweating and more particularly the feet, the axillary areas, especially the armpits, the mammary areolae and the perianal region, where the majority of the sweat glands are located, in particular the apocrine glands, or even of cerumen, in the auditory canals.

The object of the present invention is, precisely, to satisfy these needs.

Thus, according to a first aspect, the invention relates to the cosmetic use of at least an effective amount of a lysate of at least one microorganism of the genus *Bifidobacterium species,* or a fraction thereof, for preventing and/or treating body odor.

For the purposes of the present invention, the term "preventing" means reducing the risk of occurrence of the phenomenon concerned, and the term "effective amount" denotes an amount that is sufficient to obtain the expected effect.

For the purposes of the present invention, the term "body odor" is intended to denote any unpleasant odor that may be perceived as offensive, or even invalidating, by the person producing it and/or the people in his vicinity.

The body odor more particularly considered according to the invention is that directly or indirectly associated with the presence of bodily fluids secreted on an epidermis, such as sweat and sebum, especially that arising from the degradation and/or secretion of sweat or sebum, or even of cerumen in the auditory canals, and more particularly that arising from the bacterial degradation of sweat, and especially of apocrine sweat.

It is especially a case of odor noted on areas of the body that undergo heavy sweating and more particularly the axillary areas, especially the armpits, or the feet, the mammary areolae and the perianal region.

Thus, the invention relates to the cosmetic use of at least an effective amount of a lysate of at least one microorganism of the Bifidobacterium longum species or a fraction thereof, for treating the areas of the body bearing apocrine glands.

The invention also relates to the cosmetic use of at least an effective amount of a lysate of at least one microorganism of the Bifidobacterium longum species, or a fraction thereof, for treating areas of the body bearing sebaceous glands. They may especially be the auditory canals.

In particular, the use of an effective amount of a lysate of at least one microorganism of the Bifidobacterium longum species, or a fraction thereof, makes it possible to inhibit the biosynthesis by microorganisms of malodorous volatile compounds, for instance those resulting from the degradation of sweat, especially of apocrine sweat, namely C₂₋C₅ short-chain volatile acids, malodorous steroids such as the steroid 16-androstene, C₆₋C₁₂ fatty acids, such as the trans (E) isomer of 3-methyl-2-hexenoic acid (3M2H), or sulfanylalkanols.

In other words, the use of an effective amount of a lysate of at least one microorganism of the genus Bifidobacterium longum species, or a fraction thereof, makes it possible to inhibit, reduce and/or modify the degradation of sweat by the bacteria that are the cause of body odor.

The term "inhibiting" means totally or partially reducing the aspect under consideration.

The term "modifying" means changing the metabolism of the bacteria that are the cause of sweat degradation and/or the type of odorous volatile compounds generated by this bacterial degradation.

Thus, according to another of its aspects, the present invention relates to the cosmetic use of at least an effective amount of a lysate of at least one microorganism of the Bifidobacterium longum species, or a fraction thereof, for degrading, inhibiting and/or modifying the bacteria present in the auditory canals and/or in the axillary regions, the perianal area, the feet and the mammary areolae, and that are involved in the degradation of sweat and/or sebum, more particularly of apocrine sweat.

Generally, the bacteria involved in the degradation of sweat and/or sebum, more particularly of apocrine sweat, mainly consist of saprophytic micro-organisms that are non-pathogenic.

More particularly, the bacteria involved in such phenomena are of the genus *Corynebacterium,* and more particularly C. *striatum, C. mucifaciens,* especially C. *mucifaciens* DMMZ 2278, C. G2, especially C. G2CDC G5840, *C*. *jeikeium;* of the genus *Brevibacterium;* of the genus *Micrococcus,* and more particularly *M. luteus;* of the genus *Staphylococcus,* and more particularly *S. epidermis;* and of the genus *Propionibacterium,* and more particularly the bacteria of the genus *Corynebacterium.*

Mention may also be made, as microorganisms involved in such phenomena, of certain yeasts, especially of the genus *Malassezia.*

For the purposes of the present invention, the term "degrading" means partially or totally eliminating the aspect relating thereto, and, in the present case, the abovementioned microorganisms that are the cause of body odor, and in particular the bacteria of the genus *Corynebacterium.*

A lysate of the invention is thus advantageously used as deodorant active agent with regard to its efficacy in reducing and/or preventing the development of body odor.

According to another of its aspects, the present invention relates to at least an effective amount of a lysate of at least one microorganism of the *Bifidobacterium* longum species, or a fraction thereof, for the preparation of a composition, especially pharmaceutical or dermatological composition, for preventing and/or treating body odor, especially that arising from a secretion of sweat and/or sebum, and/or from the bacterial degradation of sweat, more particularly of apocrine sweat, and/or of sebum.

As stated above, since the problems of body odor are often exacerbated with chronological ageing with regard to an impairment of certain biological mechanisms, or even of nonpathological physiological hormonal changes, the compositions under consideration according to the invention prove to be most particularly advantageous for individuals generally over 50 years old.

A use in accordance with the invention may also comprise the use of a lysate of at least one microorganism of the Bifidobacterium longum species, or a fraction thereof, in combination with at least an effective amount of a related microorganism, especially a probiotic microorganism, and/or a fraction thereof; distinct from said lysate.

For the purposes of the invention, the term "distinct from said lysate" means that it is possible to distinguish within the composition either two different microorganisms or two different forms of the same microorganism. Thus, when the related microorganism is of the Bifidobacterium longum species and corresponds to the same species as that featuring the lysate required according to the invention, this related microorganism is then present in a form other than a lysate.

A use in accordance with the present invention may also comprise the use of a lysate of at least one microorganism of the Bifidobacterium longum species, or a fraction thereof, in combination with at least an effective amount of an additional active agent, which is especially intended for preventing and/or treating body odor, especially distinct from a microorganism, preferably intended for regulating and/or inhibiting sweating, for instance an antiperspirant active agent, especially as described hereinbelow.

According to one embodiment variant, said additional active agent is intended for decreasing and/or correcting excessive sweat secretion.

According to another embodiment, said additional active agent is intended for masking body odor. According to this embodiment, the additional active agent may then be chosen from fragrancing active agents.

According to one embodiment of the invention, the lysate according to the invention may be used topically.

According to another embodiment of the invention, a lysate according to the invention may be used orally.

Preferably, the lysate under consideration according to the invention is used topically.

Advantageously, the lysate under consideration according to the invention is used in the form of a formulation of deodorant type, especially a stick or an aerosol.

According to another of its aspects, the present invention relates to a cosmetic composition of stick type containing an effective amount of a lysate of at least one microorganism of the Bifidobacterium longum species, or a fraction thereof, for preventing and/or treating body odor, especially that associated with the presence of bodily fluids secreted on the epidermis, for instance sweat and sebum and/or that generated by the bacterial degradation of sweat, especially of apocrine sweat, and/or of sebum.

According to yet another of its aspects, the present invention relates to a cosmetic composition of aerosol type containing an effective amount of a lysate of at least one microorganism of the Bifidobacterium longum species, or a fraction thereof, for preventing and/or treating body odor, especially that associated with the presence of bodily fluids secreted on the epidermis, for instance sweat and sebum, and/or that generated by the bacterial degradation of sweat, especially apocrine sweat, and/or of sebum.

According to yet another of its aspects, the present invention relates to a cosmetic and/or dermatological composition that is useful for preventing and/or treating body odor, comprising, in a physiologically acceptable medium, at least an effective amount of a lysate of at least one microorganism of the Bifidobacterium longum species, or a fraction thereof, in combination with at least an effective amount of a related microorganism and/or a fraction thereof, and/or at least one additional active agent for preventing and/or treating body odor, which is especially distinct from a microorganism, preferably intended for regulating and/or inhibiting sweating, for instance an antiperspirant active agent, especially as described hereinbelow.

According to yet another of its aspects, the invention relates to a process, especially a cosmetic process, for preventing and/or treating body odor, especially that associated with the presence of bodily fluids secreted on an epidermis, for instance sweat and sebum, and/or that generated by the bacterial degradation of sweat, especially of apocrine sweat, and/or of sebum, comprising at least one step of administering to the said individual at least an effective amount of a lysate of least one microorganism of the Bifidobacterium longum species, or a fraction thereof.

As emerges from the examples that follow, the inventors have, contrary to all expectation, found that a lysate of *Bifidobacterium longum* makes it possible, via stimulation of the synthesis of proteins such as Ribonuclease 7 (Q9H1E1), dermicidin (P81605), prolactin-inducible protein (P12273), the proteins S100 A8 and A9 (P05109 and P06702), and the histone protein (Q5R2W0), to efficiently oppose colonization of the epidermis by the microorganisms responsible for unpleasant odors.

Consequently, the decrease in the population of these bacteria observed after exposing them to a lysate according to the invention makes it possible to efficiently decrease the body odor generated by the interaction of these bacteria with the bodily fluids secreted on an epidermis.

To the inventors' knowledge, this efficacy of a lysate of microorganisms of the Bifidobacterium longum species has never been described.

US 2009/0 130 073 discloses the use of microorganisms of the genus *Lactobacillus* for reducing foot odor via the inhibition of the biosynthesis of isovaleric acid by skin microorganisms.

Moreover, EP 0 043 128 discloses the use of a lysate of microorganisms, but only for the purposes of repairing the DNA of skin cells.

WO 02/28402 also discloses that probiotic microorganisms may have a beneficial effect in regulating cutaneous hypersensitivity reactions such as inflammatory and allergic reactions that arise from an immunological process.

The article *"*Probiotics in the management of atopic eczema, Clinical and Experimental Allergy 2000", Volume 30, pages 1604-1610, also reports a study concerning the effect of probiotic on infantile immune mechanisms, for instance atopic dermatitis.

US 5 756 088 discloses a dietary regime especially comprising a polyunsaturated fatty acid/or biotin, and a microorganism of the genus *Bifidobacterium,* having prophylactic and therapeutic effects on animal dermatoses.

EP 1 609 463, EP 1 642 570, EP 1 731 137 and FR 2 876 029 discloses compositions combining one or more probiotic microorganisms with a mineral cation, for treating sensitive skin.

Finally, WO 2007/015 027 proposes a combination of a probiotic microorganism with a polyunsaturated fatty acid and/or a polyunsaturated fatty acid ester, for treating sensitive skin associated with dry skin.

Consequently, none of these documents describes the use of a lysate of a microorganism of the Bifidobacterium longum species as an active agent that is useful for preventing and/or treating body odor directly or indirectly linked to the presence of bodily fluids secreted on an epidermis, such as sweat and sebum, more particularly appearing on the axillary areas, especially the armpits, or on the feet, the auditory canals, the mammary areolae and the perianal region.

### Microorganisms

As stated previously, the microorganisms of the Bifidobacterium longum species used as active agents according to the invention are used in the form of a lysate.

A lysate commonly denotes a material obtained after the destruction or dissolution of biological cells via a phenomenon known as cell lysis, thus causing the release of the intracellular biological constituents naturally contained in the cells of the microorganism under consideration.

For the purposes of the present invention, the term "lysate" is used indiscriminantly to denote all of the lysate or a fraction of said lysate obtained by lysis of the microorganism under consideration.

Thus, the invention relates to the use of a lysate of Bifidobacterium longum species Bifidobacterium species and/or a fraction thereof.

The lysate used is thus formed from all of the intracellular biological constituents and of the constituents of the cell walls and membranes.

In particular, it contains a cellular cytoplasmic fraction containing enzymes such as lactic acid dehydrogenase, phosphatases, phosphoketolases, and transaldolases and metabolites. For illustrative purposes, the constituents of cell walls are notably peptidoglycan (also known as murein or mucopeptide) and teichoic acid, and the constituents of cell membranes are glycerophospholipid compounds.

This cell lysis may be accomplished via various techniques, for instance by osmotic shock, by a heat shock, by ultrasonication, or under a mechanical stress such as centrifugation.

More particularly, this lysate can be obtained according to the technique described in US 4 464 362, and especially according to the following protocol.

A microorganism of Bifidobacterium longum species type under consideration is grown anaerobically in a suitable culture medium, for example according to the conditions described in US 4 464 362 and EP 0 043 128. When the stationary phase of growth is reached, the culture medium may be inactivated by pasteurization, for example at a temperature of 60 to 65°C for 30 minutes. The microorganisms are then collected by a conventional separation technique, for example membrane filtration, centrifuged and resuspended in a sterile NaCl solution at a physiological concentration. The lysate may be obtained by ultrasonic disintegration of such a medium so as to release the cytoplasmic fractions, the cell wall fragments and the products derived from metabolism. Next, all the components in their natural distribution are stabilized in a weakly acidic aqueous solution.

A lysate with a concentration of the order of 0.1 % to 50%, in particular from 1% to 20% and especially about 5% by weight of active material(s) relative to its total weight, is thus generally obtained.

The lysate may be used in various forms, especially in the form of a solution or in a pulverulent form, preferably in the form of a solution.

The lysate is registered under the INCI name: Bifidat ferment Lysate, under the EINECS name: Bifidobacterium longum, under the EINECS number: No. 306-168-4 and under the CAS No.: 96507-89-0.

The product sold under the name Repair Complex CLR® by the company K. Richter GmbH, and which is formed from an inactivated lysate of the species *Bifidobacterium longum,* is included in the context of the invention.

The active agent forming the lysate and belonging to the Bifidobacterium longum species may be formulated in a composition in a proportion of at least 0.0001% (expressed as dry weight), in particular in a proportion of from 0.001% to 20% and more particularly in a proportion of from 0.01% to 10% by dry weight of active material(s), relative to the total weight of the composition containing it.

The content of lysate via the topical route according to the invention may range from 0.0001% to 30% by weight, especially from 0.01 % to 15% by weight and in particular from 0.1 % to 10% by weight relative to the total weight of the composition containing it.

In the particular case where the microorganism(s) is/are formulated in compositions to be administered orally, the concentration of microorganism(s) may be adjusted so as to correspond to doses (expressed as microorganism equivalent) ranging from 5 x 10² to 10¹³ cfu/day and in particular from 10⁵ to 10¹¹ cfu/day.

According to one variant of the invention, a lysate that is suitable for use in the invention is used in combination with at least one other microorganism.

Thus, the invention relates to the use, besides a lysate of at least one microorganism of the Bifidobacterium longum lysate, of at least an effective amount of a related microorganism, especially of probiotic type, and/or a fraction thereof, and/or a metabolite thereof, distinct from said lysate.

For the purposes of the present invention, the term "probiotic microorganism" means a live microorganism which, when consumed in an adequate amount, has a positive effect on the health of its host "joint FAO/WHO Expert Consultation on Evaluation of Health and Nutritional Properties of Probiotic in Food Including Powder Milk with Live Lactic Acid Bacteria, 6 October 2001", and which may in particular improve the intestinal microbial equilibrium.

A related microorganism that is suitable for use in the invention may be chosen especially from ascomycetes such as *Saccharomyces, Yarrowia, Kluyveromyces, Torulaspora, Schizosaccharomyces pombe, Debaromyces, Candida, Pichia, Aspergillus* and *Penicillium,* bacteria of the genera *Bifidobacterium, Bacteroides, Fusobacterium, Melissococcus, Propionibacterium, Enteracoccus, Lactococcus, Staphylococcus, Peptostrepococcus, Bacillus, Pediococcus, Micrococcus, Leuconostoc, Weissella, Aerococcus, Oenococcus* and *Lactohacillus,* and mixtures thereof.

As ascomycetes that are most particularly suitable for use in the present invention, mention may be made in particular of *Yarrowia lipolytica* and *Kluyveromyces lactis,* and also *Saccharorrryces cerevisiae, Torulaspora, Schizosaccharomyces pombe, Candida* and *Pichia.*

Specific examples of related probiotic microorganisms are *Lactobacillus acidophilus, Lactobacillus alimentarius, Lactobacillus curvatus, Lactobacillus delbruckii subsp. lactis, Lactobacillus gasseri, Lactobacillus johnsonii, Lactobacillus reuteri, Lactobacillus rhamnosus (Lactobacillus GG), Lactobacillus sake, Lactobacillus paracasei, Lactococcus lactis, Streptococcus thermophilus, Staphylococcus carnosus* and *Staphylococcus xylosus,* and mixtures thereof

More particularly, they are probiotic microorganisms derived from the group of lactic acid bacteria, notably such as *Lactobacillus.* As illustrations of these lactic acid bacteria, mention may be made more particularly of *Lactobacillus johnsonii*, *Lactobacillus reuteri, Lactobacillus rhamnosus, Lactobacillus paracasei,* and mixtures thereof. As stated previously, the related microorganism may or may not be of the same species as the one forming the lysate. However, when it is of the same species, it then present in a form other than a lysate, for example in a live form.

The species that are most particularly suitable for use are *Lactobacillus johnsonii,* especially the strain deposited according to the Budapest Treaty at the Institut Pasteur (28 rue du Docteur Roux, F-75024 Paris cedex 15) under the following Accession number CNCMI-1225.

This or these related microorganism(s) may be included in a composition according to the invention in a live form, a semiactive or an inactivated form, or a dead form.

They may also be included in the form of fractions of cell components or in the form of metabolites. The microorganism(s), metabolite(s) or fraction(s) may also be introduced in the form of a powder, a liquid, a culture supernatant or a fraction thereof, optionally diluted, or optionally concentrated.

In the case where the microorganisms are formulated in a composition in a live form, the amount of live microorganisms may range from 10³ to 10¹⁵ cfu/g, in particular from 10⁵ to 10¹⁵ cfu/g and more particularly from 10⁷ to 10¹² cfu/g of microorganisms per gram of composition.

### Galenical forms

The compositions according to the invention may be in any galenical form normally available for the selected mode of administration.

The support may be of diverse nature according to the type of composition under consideration.

The cosmetic compositions of the invention may thus be in the form of a lotion, an emulsion, a cream or a fluid gel, preferably dispensed as an aerosol spray, in a pump-dispenser bottle or as a roll-on, in the form of a thick cream dispensed in a tube and in the form of a stick or a powder, and, in this regard, may contain ingredients and propellants generally used in products of this type that are well known to those skilled in the art.

For the compositions intended for topical administration, it may be an aqueous, aqueous-alcoholic or oily solution, a dispersion of the solution type or a dispersion of the lotion or serum type, an emulsion of liquid or semiliquid consistency of the milk type, obtained by dispersing a fatty phase in an aqueous phase (O/W) or conversely (W/O), or a suspension or emulsion of soft, semisolid or solid consistency, of the cream type, an aqueous or anhydrous gel, or alternatively microemulsions, microcapsules, microparticles, or vesicular dispersions of ionic and/or nonionic type.

These compositions are prepared according to the usual methods.

These compositions may especially constitute deodorant and/or antiperspirant compositions, cleansing creams, lotions, or gels for treating or caring for the skin, the axillary regions, the perianal region, the mammary areolae, the auditory canals or the feet, the major anatomical folds, or for the body (for example day cream, night cream, makeup-removing cream, foundation cream or antisun cream), protective or care body milks, after-sun milks, skincare lotions, gels or mousses, for instance cleansing or disinfecting lotions, antisun lotions, artificial tanning lotions, bath compositions, for instance shower gels or shampoos, or deodorant compositions containing a bactericidal agent, aftershave gels or lotions, or hair-removing creams.

The compositions according to the invention may also consist of solid preparations constituting soaps or cleansing bars.

Advantageously, a cosmetic composition of the invention is in the form of a deodorant or a fragrance and preferably in the form of an aerosol spray, a cream or a stick.

When the composition of the invention is an emulsion, the proportion of the fatty phase may range from 5% to 80% by weight and preferably from 10% to 50% by weight relative to the total weight of the composition. The oils, emulsifiers and coemulsifiers used in the composition in emulsion form are chosen from those conventionally used in cosmetics and/or dermatology. The emulsifier and the coemulsifier may be present in the composition in a proportion ranging from 0.3% to 30% by weight and preferably from 0.5% to 20% by weight relative to the total weight of the composition.

When the composition of the invention is an oily solution or gel, the fatty phase may represent more than 90% of the total weight of the composition.

In a known manner, galenical forms intended for topical administration may also contain adjuvants that are common in the cosmetic, pharmaceutical and/or dermatological field, such as hydrophilic or lipophilic gelling agents, hydrophilic or lipophilic active agents, preserving agents, antioxidants, solvents, fragrances, fillers, screening agents, bactericides, odor absorbers and dyestuffs.

The amounts of these various adjuvants are those conventionally used in the field under consideration, for example from 0.01% to 20% of the total weight of the composition. Depending on their nature, these adjuvants may be introduced into the fatty phase and/or into the aqueous phase.

As fatty substances that may be used in the invention, mention may be made of mineral oils, for instance hydrogenated polyisobutene and liquid petroleum jelly, plant oils, for instance a liquid fraction of shea butter, sunflower oil and apricot kernel oil, animal oils, for instance perhydrosqualene, synthetic oils, especially purcellin oil, isopropyl myristate and ethylhexyl palmitate, unsaturated fatty acids and fluoro oils, for instance perfluoropolyethers. It is also possible to use fatty alcohols, fatty acids, for instance stearic acid, and, for example, waxes, especially paraffin wax, carnauba wax and beeswax. It is also possible to use silicone compounds, for instance silicone oils, for example cyclomethicone and dimethicone, and silicone waxes, resins and gums.

As emulsifiers that may be used in the invention, examples that may be mentioned include glyceryl stearate, polysorbate 60, the mixture of cetylstearyl alcohol/cetylstearyl alcohol oxyethylenated with 33 mol of ethylene oxide, sold under the name Sinnowax AO^{®} by the company Henkel, the mixture of PEG-6/PEG-32/glycol stearate sold under the name Tefose^{®} 63 by Gattefosse, PPG-3 myristyl ether, silicone emulsifiers such as cetyldimethicone copolyol and sorbitan mono- or tristearate, PEG-40 stearate and oxyethylenated (20 OE) sorbitan monostearate.

As solvents that may be used in the invention, mention may be made of lower alcohols, for instance ethanol, isopropanol and propylene glycol.

A composition of the invention may also advantageously contain a spring and/or mineral water, chosen especially from Vittel water, waters from the Vichy basin and Roche Posay water.

Hydrophilic gelling agents that may be mentioned include carboxylic polymers such as carbomer, acrylic copolymers such as acrylate/alkyl acrylate copolymers, polyacrylamides and especially the mixture of polyacrylamides, C13-14-isoparaffin and Laureth-7 sold under the name Sepigel 305^{®} by the company SEPPIC, polysaccharides, for instance cellulose derivatives such as hydroxyalkylcelluloses and in particular hydroxypropylcellulose and hydroxyethylcellulose, natural gums such as guar gum, carob gum and xanthan gum, and clays.

Lipophilic gelling agents that may be mentioned include modified clays such as bentones, metal salts of fatty acids such as aluminum stearates and hydrophobic silica, or ethylcellulose and polyethylene.

In the case of oral use of a combination in accordance with the invention, the use of an ingestible support is preferred.

The ingestible support may be of diverse nature depending on the type of composition under consideration.

Milk, yoghurt, cheese, fermented milks, milk-based fermented products, ice creams, products based on fermented cereals, milk-based powders, baby and infant formulations, food products of candy type, chocolate, cereals, animal feed and in particular pet food, tablets, gel capsules or lozenges, oral supplements in dry form and oral supplements in liquid form are especially suitable for use as pharmaceutical or food supports.

A microorganism of the invention, and/or a fraction thereof, may moreover be formulated with the excipients and components that are common for such oral compositions or food supplements, namely, especially, fatty and/or aqueous components, humectants, thickeners, preserving agents, texture agents, taste agents and/or coating agents, antioxidants, preserving agents and dyes that are common in the food sector.

The formulating agents and excipients for an oral composition, and especially for food supplements, are known in this field and will not be the subject of a detailed description herein. Many embodiments of oral compositions and especially of food supplements are possible, for ingestion. They are formulated via usual processes for producing coated tablets, gel capsules, gels, controlled-release hydrogels, emulsions, tablets and capsules.

In particular, the microorganism according to the invention may be incorporated into any form of food supplement or enriched food, for example food bars, or compacted or loose powders. The powders may be diluted with water, in soda, dairy products or soybean derivatives, or may be incorporated into food bars.

According to one particular embodiment, the related microorganisms under consideration according to the invention, when they are in a live or inactivated form, may be formulated in compositions in an encapsulated form so as to significantly improve their survival time. In such cases, the presence of a capsule may in particular retard or prevent the degradation of the microorganism in the gastrointestinal tract.

### Additional active agents

Irrespective of the mode of administration under consideration, a lysate of the invention may advantageously be combined with at least one other additional active agent.

Thus, a topical or oral composition, or a combination according to the invention, may also contain at least one active agent for preventing and/or treating body odor, which is especially distinct from a microorganism such as those defined previously.

Such a formulation advantageously makes it possible to amplify the beneficial effects of a lysate of the invention.

An active agent for preventing and/or treating body odor that is suitable for use in the invention may especially be chosen from active agents for regulating and/or inhibiting sweating, for instance an antisweat or antiperspirant active agent.

Such agents may especially be agents that are capable of:
- reducing and/or inhibiting the activity of the apocrine and/or eccrine glands, especially the apocrine glands, or capable of reducing and/or inhibiting the flow of sweat produced, for instance aluminum and/or zirconium salts, glutaraldehyde or formaldehyde;
- reducing and/or inhibiting the degradation of sweat, especially of apocrine sweat, by the bacteria that are the cause of body odor, for instance enzymatic or nonenzymatic bactericides and more particularly triclosan or farnesol;
- inhibiting the enzymes responsible for the development of body odor;
limiting the growth of bacteria, for instance transition metal-chelating agents such as EDTA or DPTA;
- preventing the formation of and/or inhibiting and/or destroying compounds derived from the degradation of sweat that are responsible for body odor, for instance C₂-C₅ short-chain volatile fatty acids, malodorous steroids (16-androstene steroids), C₆-C₁₂ acids, or sulfanylalkanols. Such active agents may be chosen especially from arylsulfatase, 5-lipoxygenase, aminocyclase or β-glucuronidase inhibitors;
- acting on the odor per se, for instance fragrances or odor absorbers, for instance zeolites, cyclodextrins, metal oxide silicates, especially those described in US 2005/063 928, metal oxide particles modified with a transition metal, especially those described in US 20051084 464 and US 2005/084 474, aluminosilicates, especially those described in EP 1 658 863, or particles of nanometric chitosan derivatives, especially those described in US 6 916 465; and
- mixtures thereof.

The additional active agent is, for example, present in a content ranging from 0.1% to 10% by weight, preferably from 0.1% to 5% by weight and preferentially from 0.5% to 3% by weight relative to the total weight of the composition.

Besides these active agents, a composition of the invention may also comprise at least one active agent commonly used and/or permitted for a cosmetic or dermatological formulation.

As active agents that are conventionally used, mention may be made of vitamins B3, B5, B6, B8, C, E and PP, niacin, carotenoids, polyphenols, minerals and trace elements, phytoestrogens, proteins and amino acids, mono- and polysaccharides, amino sugars, phytosterols, triterpenic alcohols of plant origin, and anti-inflammatory agents.

The minerals and trace elements that are particularly used are zinc, calcium, magnesium, copper, iron, iodine, manganese, selenium or chromium (III).

Among the polyphenols, polyphenols from grape, from tea, from olive, from cocoa, from coffee, from apple, from blueberry, from elderberry, from strawberry, from cranberry and from onion are also selected in particular. Preferably, among the phytoestrogens, isoflavones in free or glycosylated form are selected, such as genisteins, daidzein, glycitein or lignans, in particular those from linseed and from *Schizandra chinensis.*

Amino acids or peptides and the proteins containing them, such as taurine, threonine, cysteine, tryptophan and methionine. The lipids preferably belong to the group of oils containing mono- and polyunsaturated fatty acids such as oleic acid, linoleic acid, α-linoleic acid, γ-linoleic acid, stearidonic acids, long-chain fish omega-3 fatty acids, such as EPA and DHA, and conjugated fatty acids derived from plants or animals, such as CLA (conjugated linoleic acid).

An antioxidant complex comprising vitamins and at carotenoid, especially a carotenoid chosen from β-carotene, lycopene, astaxanthin, zeaxanthin and lutein, flavonoids such as catechins, hesperidin, proanthocyanidins and anthocyanins, ubiquinones, coffee extracts containing polyphenols and/or diterpenes, chicory extracts, ginkgo biloba extracts, proanthocyanidin-rich grape extracts, pimento extracts, soybean extracts, other sources of flavonoids with antioxidant properties, fatty acids, prebiotics, taurine, resveratrol, selenium amino acids and glutathione precursors may be used in particular.

Among flavonoids, catechins and PCOs (procyannidol oligomers) are preferably chosen.

It may also be at least one prebiotic or a mixture of prebiotics. More particularly, these prebiotics may be chosen from oligosaccharides, produced from glucose, galactose, xylose, maltose, sucrose, lactose, starch, xylan, hemicellulose, inulin, gums of acacia type, for example, or a mixture thereof. More particularly, the oligosaccharide comprises at least one fructo-oligosaccharide. More particularly, this prebiotic may comprise a mixture of fructo-oligosaccharide and inulin.

Proteins or protein hydrolysates, amino acids, polyols, especially of C₂ C₁₀, for instance glycerol, sorbitol, butylene glycol and polyethylene glycol, urea, allantoin, sugars and sugar derivatives, water-soluble vitamins, starch, and bacterial or plant extracts such as those of *Aloe vera,* may be used more particularly as hydrophilic active agents in the topical galenical forms.

As regards the lipophilic active agents, retinol (vitamin A) and derivatives thereof, tocopherol (vitamin E) and derivatives thereof, ceramides, essential oils and unsaponifiable materials (tocotrienol, sesamine, gamma-oryzanol, phytosterols, squalenes, waxes and terpenes) may be used.

The product may advantageously be combined with active agents, such as the reference moisturizing active agents in the field of cosmetics, for instance glycerol, hyaluronic acid, urea and derivatives thereof, and also active agents that promote desquamation and peeling, such as chelating agents, jasmonic acid and derivatives thereof, particularly ER2412, reducing agents, sulfonic derivatives and particularly HEPES, amino acids, AHAs and BHAs, most particularly glycolic acid and ER195, and certain detergents, and also any odorous substance commonly used in the field of cosmetics, for instance essential oils and fragrances.

As active agents that may more particularly be combined with the lysate in an oral galenical formulation, any ingredient commonly used and/or permitted for such a mode of administration may also be considered.

Illustrations that may be mentioned include vitamins, minerals, essential lipids, trace elements, polyphenols, flavonoids, phytoestrogens, antioxidants such as lipoic acid and coenzyme Q10, carotenoids, probiotics, prebiotics, proteins and amino acids, monosaccharides and polysaccharides, amino sugars, phytosterols and triterpenic alcohols of plant origin, or chlorophyll.

They are, in particular, vitamins A, C, D, E and PP and group B vitamins. Among the carotenoids, β-carotene, lycopene, lutein, zeaxanthin and astaxanthin are preferably chosen. The minerals and trace elements particularly used are zinc, calcium, magnesium, copper, iron, iodine, manganese, selenium and chromium (III). Among the polyphenols, polyphenols from grape, from tea, from olive, from cocoa, from coffee, from apple, from blueberry, from elderberry, from strawberry, from cranberry and from onion are also selected in particular. Preferably, among the phytoestrogens, isoflavones in free or glycosylated form are selected, such as genisteins, daidzein, glycitein or lignans, in particular those from linseed and from *Schizandra chanensis.* Amino acids or peptides and the proteins containing them, such as taurine, threonine, cysteine, tryptophan and methionine. The lipids preferably belong to the group of oils containing mono- and polyunsaturated fatty acids such as oleic acid, linoleic acid, α-linoleic acid, γ-linoleic acid, stearidonic acids, long-chain fish omega-3 fatty acids, such as EPA and DHA, and conjugated fatty acids derived from plants or animals, such as CLA (conjugated linoleic acid). Essential oils, for instance citral (or lemonal), linalyl acetate, geraniol (or rhodinol) or menthol, may also be mentioned.

A cosmetic treatment process of the invention may especially be performed by administering the cosmetic and/or dermatological compositions or combinations as defined above, according to the usual technique for using these compositions.

A cosmetic process according to the invention may thus be performed by topical or oral administration, for example daily, of the lysate under consideration according to the invention.

A process according to the invention may comprise a single administration. According to another embodiment, the administration is repeated, for example two to three times daily over a day or more and generally over a prolonged period of at least four weeks, or even four to 15 weeks, with, where appropriate, one or more periods of stoppage.

In the description and in the examples that follow, unless otherwise mentioned, the percentages are weight percentages and the ranges of values given in the form "between... and..." include the stated lower and upper limits.

The ingredients are mixed together, before being formed, in the order and under conditions that may readily be determined by a person skilled in art.

Furthermore, treatment combinations optionally with oral or topical forms may be envisioned, in order to complement or to reinforce the activity of the lysate as defined by the invention.

Thus, according to one aspect of reinforcement of the activity of said lysate, a topical treatment may be envisaged with a composition containing at least one lysate of at least one microorganism of the Bifidobacterium longum species combined with an oral or topical composition optionally containing at least one other probiotic microorganism or other probiotics, in a dead form, a live form or a semiactive form.

According to one aspect of complementation of the activity of said lysate, a topical treatment may also be envisioned with a composition containing at least one lysate of at least one microorganism of the Bifidobacterium longum species combined with any topical composition, for example for caring for and/or treating keratin materials, for instance skincare compositions such as shower gels, or more specific topical compositions intended, for example, for preventing and/or treating chronological and/or photoinduced ageing of the skin, and optionally combined with skin dryness.

The content and nature of the ingredients used in the compositions of the invention are adjusted by a person skilled in art so as not to substantially affect the properties required for the compositions of the invention.

The examples below are presented as nonlimiting illustrations of the field of the invention.

### Examples

### Example 1: Lotion for combating body odor

**Compounds**

| INCI Bifidat ferment Lysate* | |
|---|---|
| Anti-inflammatory agent | |
| Antioxidant | |
| Isopropanol | 40.0 |
| Preserving agent | |
| Water | qs 100 |

| | |
|---|---|
| *This is the lysate registered under the name INCI Bifidat ferment Lysate, and used in the form of a formulation containing 5% by weight of active agent **amount expressed as total product | |

### Example 2: Milk for combating body odor

| **Compounds** | **% by weight** |
|---|---|
| INCI Bifidat ferment Lysate* | 5.00** |
| Glyceryl stearate | 1.00 |
| Cetylstearyl oil/cetylstearyl oil oxyethylenated with 30 mol OE | |
| (Sinnowax AO^{®} sold by the company Henkel) | 3.00 |
| Cetyl alcohol | 1.00 |
| Dimethicone (DC 200 Fluid^{®} sold by the | 1.00 |
| Liquid petroleum jelly | 6.00 |
| Isopropyl myristate | 3.00 |
| Antioxidant | 0.05 |
| Glycerol | 20.00 |
| Preserving agent | 0.30 |
| Water | qs 100 |

| | |
|---|---|
| This is the lysate registered under the name INCI Bifidat ferment Lysate, and used in the form of a formulation containing 5% by weight of active agent ** Amount expressed as total product | |

### Example 3: Evaluation of the effects of a lysate of Bifidobacterium longum on the population of microorganism responsible for body odor

The test product is a lysate of *Bifidobacterium longum* (INCI: Bifidat ferment Lysate) as a disintegrated (by ultrasonication) suspension in a weakly acidic aqueous medium.

The active agent is tested alone in a double-blind randomized study. 66 women with dry skin were divided into two groups, namely a "placebo" group (n = 33, group A) and a *"Bifidobacterium longum* lysate" group (INCI: Bifidat ferment Lysate) (n = 33, group B).

The treatments were applied topically for 58 days, the active agent being formulated at 10% of the test formulation.

The support formula is an O/W (demineralized) emulsion Arlacel/Myrj^{®} containing 5% parleam, 15% cyclopentasiloxane, 3% glycerol and 2% petroleum jelly.

In the placebo formula, the absence of lysate is compensated for with water.

The individuals were evaluated on D1, D29, D43 and D57.

The change in the various cutaneous markers was studied by differential proteomics on samples of isolated *stratum corneum.*

A skin sample is taken at times D1, D29, D43 and D57 by varnish stripping, so as to remove only part of the *stratum corneum,* i.e. not more than 4 to 5 layers of *stratum corneum.*

A 41 µm nylon filter gauze of Millipore NY41 type is applied to a predefined area of the left leg. A reference transparent varnish 614254/T.D. comprising: nitrocellulose 6.86 g; isopropanol 2.94 g; hypoallergenic alkyl resin 7.35 g; acetyl tributyl citrate 7.7 g; ethyl acetate 75.15 g; is spread on using a brush (15 mm) and then left to dry for 15 minutes. The nylon gauze is then recovered using Brucelles tweezers by pulling off the varnish strip in a single motion.

The varnish strippings are stored flat at -20°C in plastic bags.

These skin samples (varnish stripping of *stratum corneum)* were then analyzed by proteomics using the "isobaric labeling" technique, to evaluate the expression of various proteins.

This "isobaric labeling" technique or iTRAQ is based on the labeling of tryptic peptides with a series of reagents that are said to be isobaric since they all have a molecular mass of 145 Da, and form a covalent bond with the primary amines of the amino-terminal end or of the side chain of lysine residues.

The labeled peptides are detected by mass spectrometry with the intrinsic mass of the peptide plus 145 Da coming from the reagent. In the step of fragmentation of the peptide, the contribution of each of the reagents is assessed by release (fragments) having different specific masses.

Such a method is more specifically described by Zieske (J. Exp. Bot., 2006, 57: 1501) or Wiese et al. (Proteomics, 2007, 7: 340).

The results of the proteomics analysis showed that the lysate of *Bifidobacterium longum* stimulates the expression of various antimicrobial defense proteins of the epidermis such as ribonuclease 7 (access No. Uniref Q9H1E1), dermicidin (P81605), "prolactin-inducible protein" (P12273), the proteins S100 A8 and A9 (P05109 and P06702), and the histone protein (Q5R2W0).

The demonstration of stimulation of the abovementioned proteins is an indication of the potential decrease in colonization by certain microorganisms, in particular by the bacteria of the genus *Corynebacterium* and more particularly *C*. *striatum, C. mucifaciens,* especially *C*. *mucifaciens* DMMZ 2278, *C*. G2, especially *C*. G2CDC G5840 and *C*. *jeikeium;* of the genus *Brevibacterium;* of the genus *Micrococcus,* and more particularly *M. luteus;* of the genus *Staphylococcus,* and more particularly *S*. *epidermis;* and of the genus *Propionibacterium,* but also by certain yeasts, especially of the genus *Malassezia,* which are the main microorganisms responsible, via their metabolism, for the development of unpleasant body odor.

A lysate of the invention thus contributes toward decreasing unpleasant body odor via efficacy with regard to the colonies of microorganisms involved in the degradation of the bodily fluids secreted on the epidermis, in particular sweat and/or sebum, the cause of the formation and release of malodorous volatile compounds.

## Claims

1. The cosmetic use of at least an effective amount of a lysate of at least one microorganism of the *Bifidobacterium longum* species, or a fraction thereof, for preventing and/or treating body odor.

2. The use according to claim 1, in which the body odor is directly or indirectly linked to the presence of bodily fluids secreted on the epidermis, such as sweat and sebum, especially those arising from the degradation and/or secretion of sweat or sebum, or even cerumen in the auditory canal, and more particularly those arising from the bacterial degradation of sweat, and especially of apocrine sweat.

3. The cosmetic use of at least an effective amount of a lysate of at least one microorganism of the *Bifidobacterium longum* species, or a fraction thereof, as a deodorant active agent.

4. The use according to any one of the preceding claims, for degrading, inhibiting and/or modifying the bacteria present in the auditory canals and/or on the axillary regions, the perianal area, the feet and the mammary areolae and involved in the degradation of sweat and/or sebum, more particularly of apocrine sweat.

5. The use according to any one of the preceding claims, in which the lysate is that registered under the INCI name: Bifidat ferment Lysate.

6. The use according to any one of the preceding claims, in which said lysate comprises at least 0.0001% by weight, preferably from 0.001% to 20% by weight and in particular from 0.01% to 10% by dry weight of active material(s), relative to the total weight of the composition containing it.

7. The use according to any one of the preceding claims, in which said lysate is in combination with at least an effective amount of a related microorganism, especially a probiotic microorganism, and/or a fraction thereof, distinct from said lysate.

8. The use according to the preceding claim, in which said related microorganism is chosen from ascomycetes such as *Saccharomyces, Yarrowia, Kluyveromyces, Torulaspora, Schizosaccharomyces pombe, Debaromyces, Candida, Pichia, Aspergillus* and *Penicillium,* bacteria of the genus *Bifidobacterium, Bacteroides, Fusobacterium, Melissococcus, Propianibacterium, Enterococcus, Lactococcus, Staphylococcus, Peptostrepococcus, Bacillus, Pediococcus, Micrococcus, Leuconostoc, Weissedla, Aerococcus, Oenococcus* and *Lactobacillus,* and mixtures thereof.

9. The use according to any one of the preceding claims, in which said lysate is in combination with at least an effective amount of an additional active agent for preventing and/or treating body odor, which is preferably intended for regulating and/or inhibiting sweating.

10. The use according to any one of the preceding claims, **characterized in that** said lysate is used in the form of a formulation of deodorant type, especially a stick or an aerosol.

11. A cosmetic composition that is useful for preventing and/or treating body odor, comprising, in a physiologically acceptable medium, at least an effective amount of a lysate of at least one microorganism of the *Bifidobacterium longum* species, or a fraction thereof, in combination with at least an effective amount of a related microorganism and/or a fraction thereof, and/or at least one additional active agent for preventing and/or treating body odor, which is preferably intended to regulate and/or inhibit sweating, and better still an antiperspirant active agent.

12. A cosmetic process for preventing and/or treating body odor in an individual, especially that associated with the presence of bodily fluids secreted on an epidermis, for instance sweat and sebum, and/or of that generated by the bacterial degradation of sweat, especially of apocrine sweat, and/or of sebum, comprising at least one step of administering to the said patient at least an effective amount of a lysate of at least one microorganism of the *Bifidobacterium longum* species, or a fraction thereof.

## Patentansprüche

1. Kosmetische Verwendung von mindestens einer wirksamen Menge eines Lysats von mindestens einem Mikroorganismus der Spezies *Bifidobacterium longum* oder einer Fraktion davon zur Verhinderung und/oder Behandlung von Körpergeruch.

2. Verwendung nach Anspruch 1, wobei der Körpergeruch direkt oder indirekt mit der Gegenwart von auf der Oberhaut sezernierten Körperfluiden zusammenhängt, wie Schweiß und Talg, insbesondere denjenigen, die aus der Zersetzung und/oder der Sekretion von Schweiß oder Talg herrühren, oder auch Ohrenschmalz im Gehörgang, und insbesondere denjenigen, die durch den bakteriellen Abbau von Schweiß und insbesondere apokrinem Schweiß entstehen.

3. Kosmetische Verwendung von mindestens einer wirksamen Menge eines Lysats von mindestens einem Mikroorganismus der Spezies *Bifidobacterium longum* oder einer Fraktion als Deodorant-Wirkstoff.

4. Verwendung nach einem der vorangehenden Ansprüche zum Abbau, zur Hemmung und/oder Modifizierung der Bakterien, die im Gehörgang und/oder in den Achselhöhlen, dem Perianalbereich, den Füßen und im Brustwarzenhof vorhanden und an der Zersetzung von Schweiß und/oder Talg, insbesondere apokrinem Talg, beteiligt sind.

5. Verwendung nach einem der vorangehenden Ansprüche, wobei das Lysat, dasjenige ist, das unter dem INCI-Namen Bifidat Ferment Lysate registriert ist.

6. Verwendung nach einem der vorangehenden Ansprüche, wobei das Lysat mindestens 0,0001 Gew.-%, vorzugsweise 0,001 Gew.-% bis 20 Gew.% und insbesondere 0,01 Gew.-% bis 10 Gew.-% Trockengewicht an wirksamem Material (wirksamen Materialien) relativ zum Gesamtgewicht der Zusammensetzung, die es enthält, umfasst.

7. Verwendung nach einem der vorangehenden Ansprüche, wobei das Lysat in Kombination mit mindestens einer wirksamen Menge eines verwandten Mikroorganismus, insbesondere eines probiotischen Mikroorganismus und/oder einer Fraktion davon, die sich von dem Lysat unterscheidet, vorhanden ist.

8. Verwendung nach einem der vorangehenden Ansprüche, wobei der verwandte Mikroorganismus ausgewählt wird aus Ascomycetes wie *Saccaromyces, Yarrowia, Kluyveramyces, Torulaspora, Schizosaccaromyces pombe, Debaromyces, Candida, Pichia, Aspergillus und Penicillium,* Bakterien der Gattung *Bifidobaceterium, Bacteroides, Fusobacterium, Melissococcus, Propionibacterium, Enterococcus, Lactococcus, Staphylococcus, Peptastreptococcus, Bacillus, Pediococcus, Microcaccus, Leuconostoc, Weissella, Aerococcus, Oenococcus* und *Lactobacillus,* und Gemischen davon.

9. Verwendung nach einem der vorangehenden Ansprüche, wobei das Lysat in Kombination mit mindestens einer wirksamen Menge eines zusätzlichen Wirkstoffs zur Verhinderung und/oder Behandlung von Körpergeruch vorhanden ist, die vorzugsweise zur Regulierung und/oder Hemmung des Schwitzens gedacht ist.

10. Verwendung nach einem der vorangehenden Ansprüche, **dadurch gekennzeichnet, dass** das Lysat in der Form einer Formulierung von Typ eines Deodorants, insbesondere eines Stifts oder eines Aerosols vorhanden ist.

11. Kosmetische Zusammensetzung, die zur Verhinderung und/oder Behandlung von Körpergeruch geeignet ist, in einem physiologisch verträglichen Medium, mindestens einer wirksamen Menge eines Lysats von mindestens einem Mikroorganismus der Spezies *Bifidobacterium longum* oder einer Fraktion davon in Kombination mit mindestens einer wirksamen Menge eines verwandten Mikroorganismus und/oder einer Fraktion davon, und/oder mindestens einem zusätzlichen Wirkstoff zur Verhinderung und/oder Behandlung von Körpergeruch, der vorzugsweise zur Regulierung und/oder Hemmung des Schwitzens gedacht ist, noch besser einem Antitranspirant-Wirkstoff

12. Kosmetisches Verfahren zur Verhinderung und/oder Behandlung von Körpergeruch in einem Individuum, insbesondere demjenigen, der mit der Gegenwart von auf der Oberhaut sezernierten Körperfluiden zusammenhängt, beispielsweise Schweiß und Talg, insbesondere und/oder demjenigen, der durch die bakterielle Zersetzung von Schweiß, insbesondere apokrinem Schweiß und/oder Talg erzeugt wird, umfassend mindestens einen Schritt der Verabreichung an den Patienten mindestens einer wirksamen Menge eines Lysats von mindestens einem Mikroorganismus der Spezies *Bifidobacterium longum* oder einer Fraktion davon.

## Revendications

1. Utilisation cosmétique d'au moins une quantité efficace d'un lysat d'au moins un microorganisme de l'espèce *Bifidobacterium longum,* ou d'une fraction de celui-ci, pour prévenir et/ou traiter les odeurs corporelles.

2. Utilisation selon la revendication 1, dans laquelle les odeurs corporelles sont directement ou indirectement liées à la présence de fluides corporels sécrétés sur l'épiderme, tels que la sueur et le sébum, en particulier ceux provenant de la dégradation et/ou de la sécrétion de la sueur ou du sébum, ou même du cérumen du canal auditif, et plus particulièrement ceux provenant de la dégradation bactérienne de la sueur, et en particulier de la sueur apocrine.

3. Utilisation cosmétique d'au moins une quantité efficace d'un lysat d'au moins un microorganisme de l'espèce *Bifidobacterium longum,* ou d'une fraction de celui-ci, en tant qu'agent actif déodorant.

4. Utilisation selon l'une quelconque des revendications précédentes, pour la dégradation, 1'inhibition et/ou la modification des bactéries présentes dans les canaux auditifs et/ou sur les régions axillaires, la zone péri-anale, les pieds et l'aréole mammaire et jouant un rôle dans la dégradation de la sueur et/ou du sébum, plus particulièrement de la sueur apocrine.

5. Utilisation selon l'une quelconque des revendications précédentes, dans laquelle le lysat est celui enregistré sous le nom INCI : lysat de ferment Bifidat.

6. Utilisation selon l'une quelconque des revendications précédentes, dans laquelle ledit lysat comprend au moins 0,0001 % en poids, de préférence de 0,001 % à 20 % en poids et en particulier de 0,01 % à 10 % en poids sec de substance(s) active (s), par rapport au poids total de la composition le contenant.

7. Utilisation selon l'une quelconque des revendications précédentes, dans laquelle ledit lysat est en combinaison avec au moins une quantité efficace d'un microorganisme apparenté, en particulier d'un microorganisme probiotique, et/ou d'une fraction de celui-ci, distinct dudit lysat.

8. Utilisation selon la revendication précédente, dans laquelle ledit microorganisme apparenté est choisi parmi les ascomycètes tels que *Saccharomyces, Yarrowia, Kluyveromyces, Torulaspora, Schizosaccharomyces pombe, Debaromyces, Candida, Pichia, Aspergillus* et Penicillium, les bactéries du genre *Bifidobacterium, Bacteroides, Fusobacterium, Melissococcus, Propionibacterium, Enterococcus, Lactococcus, Staphylococcus, Peptostrepococcus, Bacillus, Pediococcus, Micrococcus, Leuconostoc, Weissella, Aerococcus, Oenococcus* et *Lactobacillus,* et leurs mélanges.

9. Utilisation selon l'une quelconque des revendications précédentes, dans laquelle ledit lysat est en combinaison avec au moins une quantité efficace d'un agent actif additionnel pour prévenir et/ou traiter les odeurs corporelles, qui est de préférence destinée à réguler et/ou inhiber la transpiration.

10. Utilisation selon l'une quelconque des revendications précédentes, **caractérisée en ce que** ledit lysat est utilisé sous la forme d'une formulation de type déodorant, en particulier d'un bâton ou d'un aérosol.

11. Composition cosmétique qui est utile pour prévenir et/ou traiter les odeurs corporelles, comprenant, dans un milieu physiologiquement acceptable, au moins une quantité efficace d'un lysat d'au moins un microorganisme de l'espèce *Bifidobacterium longum,* ou d'une fraction de celui-ci, en combinaison avec au moins une quantité efficace d'un microorganisme apparenté et/ou d'une fraction de celui-ci, et/ou d'au moins un agent actif additionnel pour prévenir et/ou traiter les odeurs corporelles, qui est de préférence destinée à réguler et/ou inhiber la transpiration, et plus préférablement un agent actif transpirant.

12. Procédé cosmétique pour prévenir et/ou traiter les odeurs corporelles chez une personne, en particulier associées à la présence de fluides corporels sécrétés sur un épiderme, par exemple la sueur et le sébum, et/ou de ceux générés par la dégradation bactérienne de la sueur, en particulier de la sueur apocrine, et/ou du sébum, comprenant au moins une étape d'administration audit patient d'au moins une quantité efficace d'un lysat d'au moins un microorganisme de l'espèce *Bifidobacterium longum,* ou d'une fraction de celui-ci.
